# EUROPEAN PATENT APPLICATION

(11) **EP 1 743 637 A1**
(43) Date of publication of application: **17.01.2007**
(21) Application number: 05384014.6
(22) Date of filing: 15.07.2005
(51) Int. Cl.: A61K 31/415, A61P 3/04, A61P 3/10

(54) **Use of substituted pyrazole compounds and combinations thereof for the treatment of the metabolic syndrome**

(71) Applicant: LABORATORIOS DEL DR. ESTEVE, S.A., 08041 Barcelona (ES)
(72) Inventor: Buschmann, Helmut Heinrich, 08960 Sant Just Desvern, Barcelona (ES)

(57) **Abstract**

The present invention relates to the use of substituted pyrazole compounds for the treatment of metabolic syndrome in humans and animals and to corresponding medicaments.

## Description

The present invention relates to the use of substituted pyrazole compounds for the treatment of metabolic syndrome in humans and animals and corresponding medicaments.

The metabolic syndrome is a widespread disease or health state involving high risks for a significant part of the population making it interesting to treat and to take precautions in way of a prophylaxis against or the avoid the metabolic syndrome.

Thus, it was an object of the present invention to provide compounds for use as active substances in medicaments, which are suitable for the treatment or prophylaxis of the metabolic syndrome.

Said object was achieved by using the substituted pyrazole compounds of general formula I and II given below and corresponding salts and solvates thereof.

It has been found that these compounds have a marked effect on the metabolic syndrome.

Thus, in one of its aspects the present invention relates to a medicament comprising at least one substituted pyrazole compounds of general formula I, 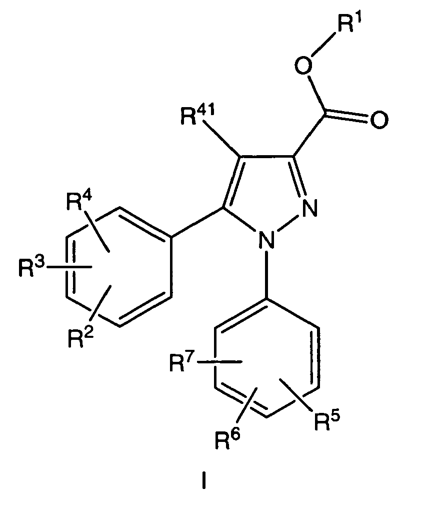 wherein
R¹ represents hydrogen or a linear or branched, substituted or unsubstituted, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵, R⁶ and R⁷ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
R⁴¹ represents hydrogen, a linear or branched, substituted or unsubstituted, saturated or unsaturated C₁₋₆-alkyl group, a linear or branched, substituted or unsubstituted, saturated or unsaturated C₁₋₆-alkoxy group, a halogen atom, CH₂F, CHF₂, CF₃, CN, OH, NO₂, -(C=O)-R^{10a}, SH, SR^{10a}, SOR^{10a}, NH₂, NHR^{10a}, NR^{10a}R^{11a}, -(C=O)-NH₂, -(C=O)-NHR^{10a} and -(C=O)-NR^{10a}R^{11a}, whereby R^{10a} and optionally R^{11a} for each substituent independently represent linear or branched C₁₋₆ alkyl;
   optionally in form of a corresponding N-oxide thereof, or a corresponding salt thereof, or a corresponding solvate thereof,
   and optionally one or more pharmaceutically acceptable excipients.

These compounds have a surprising effect on the metabolic syndrome.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

In the context of this invention, alkyl and cycloalkyl radicals are understood as meaning saturated and unsaturated (but not aromatic), branched, unbranched and cyclic hydrocarbons, which can be unsubstituted or mono- or polysubstituted. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also CHF₂, CF₃ or CH₂OH) as well as pyrazolinone, oxopyrazolinone, [1,4]-dioxane or dioxolane.

Here, in connection with alkyl and cycloalkyl - unless expressly defined otherwise - the term substituted in the context of this invention is understood as meaning replacement of at least one hydrogen radical by F, Cl, Br, I, NH₂, SH or OH, "polysubstituted" radicals being understood as meaning that the replacement takes effect both on different and on the same atoms several times with the same or different substituents, for example three times on the same C atom, as in the case of CF₃, or at different places, as in the case of -CH(OH)-CH=CH-CHCl₂. Particularly preferred substituents here are F, Cl and OH. In respect of cycloalkyl, the hydrogen radical can also be replaced by OC₁₋₃-alkyl or C₁₋₃-alkyl (in each case mono- or polysubstituted or unsubstituted), in particular methyl, ethyl, n-propyl, i-propyl, CF₃, methoxy or ethoxy.

The term (CH₂)₃₋₆ is to be understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a CF₃, a CN, an NO₂, an NRR, a C₁₋₆-alkyl (saturated), a C₁₋₆-alkoxy, a C₃₋₈-cycloalkoxy, a C₃₋₈-cycloalkyl or a C₂₋₆-alkylene, with R meaning hydrogen or C₁₋₆-alkyl.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions. It especially includes physiologically acceptable salts, which is to be used equivalently to pharmacologically acceptable salts.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of:
hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound according to the invention in which this compound has attached to it via non-covalent binding another molecule (most likely a polar solvent) especially alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The invention also covers the use of any prodrug of the compounds described for the invention. Examples of well known methods of producing a prodrug of a given acting compound are known to those skilled in the art and can be found e.g. in Krogsgaard-Larsen et al., Textbook of Drugdesign and Discovery, Taylor & Francis (April 2002).

The process to acquire the compounds according to the general formula I, II, and X as well as suitable dose ranges for the compound according to general formula X are described in or can easily be derived from EP 576 357 A1, EP 656 354 A1 and US 5,624,941 as well as EP969832 A1 and US 6,893,659 B2. All of these patents and applications are included here by reference.

As an addition to that substituted pyrazole compounds of general formula I or II, which comprise nitrogen-atom containing saturated, unsaturated or aromatic rings may be obtained in the form of their N-oxides by methods well known to those skilled in the art.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice., in particular with an enteric coating.

The mentioned formulations will be prepared using standard methods such as those described or referred to in the Spanish and US Pharmacopeias and similar reference texts.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

In a preferred embodiment of the medicament according to the invention for the compound comprised according to general formula I R⁴¹ represents hydrogen or a linear or branched, substituted or unsubstituted, saturated or unsaturated C₁₋₆-alkyl group; preferably hydrogen or C₁₋₃-alkyl; most preferably hydrogen, methyl or ethyl.

In a preferred embodiment of the medicament according to the invention for the compound comprised according to general formula I at least one of R², R³ or R⁴ represents hydrogen, while at least one of R², R³ or R⁴ is different from hydrogen.

In a preferred embodiment of the medicament according to the invention for the compound comprised according to general formula I R², R³ and R⁴ independently of each other represent hydrogen, a linear or branched C₁₋₆-alkyl group, a linear or branched C₁₋₆-alkoxy group, a halogen atom, or CF₃; preferably hydrogen, chlorine, bromine, C₁₋₃-alkyl, C₁₋₃-alkoxy, CF₃ or NO₂; most preferably R², R³ and R⁴ independently of each other represent hydrogen, methyl, ethyl, F, Cl, Br and CF₃.

In a preferred embodiment of the medicament according to the invention for the compound comprised according to general formula I R⁵, R⁶ and R⁷ independently of each other represent hydrogen, a linear or branched C₁₋₆-alkyl group, a linear or branched C₁₋₆-alkoxy group, a halogen atom, or CF₃; preferably hydrogen, chlorine, bromine, C₁₋₃-alkyl, C₁₋₃-alkoxy, CF₃ or NO₂.

In a preferred embodiment of the medicament according to the invention for the compound comprised according to general formula I R⁷ represents hydrogen.

In a preferred embodiment of the medicament according to the invention for the compound comprised according to general formula I R⁵ and R⁶ independently of each other represent a linear or branched C₁₋₆-alkyl group, a halogen atom, or CF₃, preferably R⁵ and R⁶ independently of each other represent methyl, ethyl, F, Cl, Br and CF₃.

In a preferred embodiment of the medicament according to the invention for the compound comprised according to general formula I R² represents a chlorine or a bromine atom in the 4-position of the phenyl ring, while R³ and R⁴ represent hydrogen.

In a preferred embodiment of the medicament according to the invention for the compound comprised according to general formula I R⁵ and R⁶ each represent a chlorine atoms in the 2- and 4-position of the phenyl ring, while R⁷ represents hydrogen.

In a preferred embodiment of the medicament according to the invention for the compound comprised according to general formula I R¹ represents hydrogen, methyl or ethyl, preferably hydrogen.

In a preferred embodiment of the medicament according to the invention for the compound comprised according to general formula I is selected from a compound of general formula II 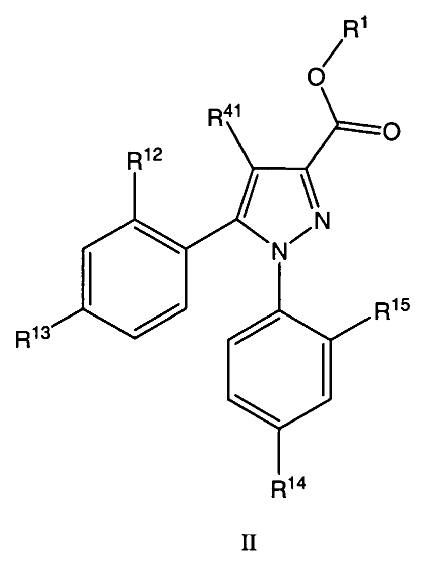 wherein
R¹ represents hydrogen or a linear or branched, substituted or unsubstituted, saturated or unsaturated C₁₋₄-alkyl group,
R¹² in a compound according to general formula II represents hydrogen, a linear or branched, substituted or unsubstituted, saturated or unsaturated C₁₋₆-alkyl group; a linear or branched, substituted or unsubstituted, saturated or unsaturated C₁₋₆-alkoxy group; a halogen atom, CN, OH, NO₂, SH, NH₂,
R¹³ represents a linear or branched, substituted or unsubstituted, saturated or unsaturated C₁₋₆-alkyl group; a linear or branched, substituted or unsubstituted, saturated or unsaturated C₁₋₆-alkoxy group; a halogen atom, CN, OH, NO₂, SH, NH₂,
R¹⁴ or R¹⁵ independently of each other represent a linear or branched, substituted or unsubstituted, saturated or unsaturated C₁₋₆-alkyl group; a linear or branched, substituted or unsubstituted, saturated or unsaturated C₁₋₆-alkoxy group; a halogen atom, CN, OH, NO₂, SH, NH₂,
R⁴¹ represents hydrogen, a linear or branched, substituted or unsubstituted, saturated or unsaturated C₁₋₆-alkyl group; a linear or branched, substituted or unsubstituted, saturated or unsaturated C₁₋₆-alkoxy group; a halogen atom, CN, OH, NO₂, SH, NH₂;
   optionally in form of a corresponding N-oxide thereof, or a corresponding salt thereof, or a corresponding solvate thereof.

In a preferred embodiment of the medicament according to the invention for the compound comprised according to general formula II R⁴¹ represents hydrogen or a linear or branched C₁₋₆-alkyl group, preferably hydrogen, methyl or ethyl.

In a preferred embodiment of the medicament according to the invention for the compound comprised according to general formula II R¹² represent hydrogen, a linear or branched C₁₋₆-alkyl group, a halogen atom, CH₂F, CHF₂, CF₃, CN, OH, NO₂, SH, or NH₂, preferably R¹² represents hydrogen, methyl, ethyl, F, Cl, Br and CF₃.

In a preferred embodiment of the medicament according to the invention for the compound comprised according to general formula II R¹³ represents hydrogen, a linear or branched C₁₋₆-alkyl group, a halogen atom, CH₂F, CHF₂, CF₃, CN, OH, NO₂, SH, or NH₂, preferably R¹³ represents hydrogen, methyl, ethyl, F, Cl, Br and CF₃.

In a preferred embodiment of the medicament according to the invention for the compound comprised according to general formula II R¹⁴, and R¹⁵ independently of each other represent a linear or branched C₁₋₆-alkyl group, a halogen atom, or CF₃, preferably R¹⁴ and R¹⁵ independently of each other represent methyl, ethyl, F, Cl, Br and CF₃.

In a preferred embodiment of the medicament according to the invention for the compound comprised according to general formula II R¹² represents hydrogen.

In a preferred embodiment of the medicament according to the invention for the compound comprised according to general formula II R¹³ represents Cl or Br.

In a preferred embodiment of the medicament according to the invention for the compound comprised according to general formula II R¹⁴ and R¹⁵ each represent Cl.

In a preferred embodiment of the medicament according to the invention for the compound comprised according to general formula II R¹ represents hydrogen, methyl or ethyl, preferably hydrogen.

In a preferred embodiment of the medicament according to the invention the compound comprised according to general formula I or II is selected from the group consisting of:
- 1-(2,4-dichlorophenyl)-5-(4-chlorophenyl)-4-methyl-1H-pyrazole-3-carboxylic acid,
- 1-(2,4-dichlorophenyl)-5-(4-bromophenyl)-4-methyl-1H-pyrazole-3-carboxylic acid,
- 1-(2,4-dichlorophenyl)-5-(4-chlorophenyl)-4-ethyl-1 H-pyrazole-3-carboxylic acid,
- 1-(2,4-dichlorophenyl)-5-(4-bromophenyl)-4-ethyl-1H-pyrazole-3-carboxylic acid
- 1-(2,4-dichlorophenyl)-5-(4-chlorophenyl)-1H-pyrazole-3-carboxylic acid, or
- 1-(2,4-dichlorophenyl)-5-(4-bromophenyl)-1 H-pyrazole-3-carboxylic acid,
   optionally in the form of a corresponding N-oxide, a corresponding salt or a corresponding solvate.

In another preferred embodiment of the medicament according to the invention the medicament comprises a combination consisting of at least one compound according to either of general formulas I or II and at least one substituted pyrazole compound according to general formula X, 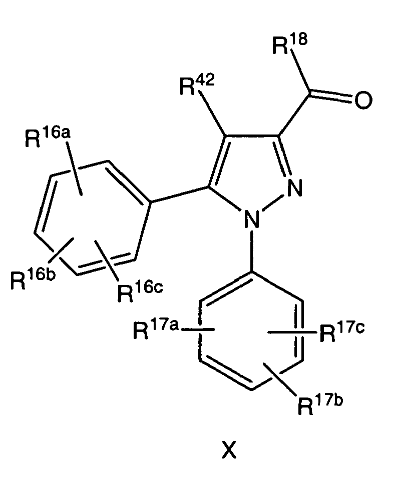 wherein
R^{16a}, R^{16b} and R^{16c} independently of one another represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁶, SH, SR²⁶, SOR²⁶, SO₂R²⁶, NH₂, NHR²⁶, NR²⁶R²⁷, -(C=O)-NH₂, -(C=O)-NHR²⁶ or -(C=O)-NR²⁶R²⁷ whereby R²⁶ and R²⁷ for each substituent independently represent linear or branched C₁₋₆ alkyl,
R^{17a}, R^{17b} and R^{17c} independently of one another represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁶, SH, SR²⁶, SOR²⁶, SO₂R²⁶, NH₂, NHR²⁶, NR²⁶R²⁷, -(C=O)-NH₂, -(C=O)-NHR²⁶ or -(C=O)-NR²⁶R²⁷ whereby R²⁶ and R²⁷ for each substituent independently represent linear or branched C₁₋₆ alkyl,
R¹⁸ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system, or an ― NR¹⁹R²⁰-moiety,
R¹⁹ and R²⁰, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R²¹-moiety, or an ― NR²²R²³-moiety, with the proviso that R¹⁹ and R²⁰ do not identically represent hydrogen,
R²¹ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R²² and R²³, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
R⁴² in the compound according to general formula X represents hydrogen; a linear or branched, saturated or unsaturated, substituted or unsubstituted C₁₋₆-alkyl group; a linear or branched, saturated or unsaturated, substituted or unsubstituted C₁₋₆-alkoxy group, a halogen atom, CH₂F, CHF₂, CF₃, CN, OH, NO₂, -(C=O)-R²⁴, SH, SR²⁴, SOR²⁴, NH₂, NHR²⁴, NR²⁴R²⁵, -(C=O)-NH₂, - (C=O)-NHR²⁴ and -(C=O)-NR²⁴R²⁵, whereby R²⁴ and optionally R²⁵ for each substituent independently represent linear or branched C₁₋₆ alkyl;
   optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In a preferred embodiment of the medicament according to the invention for the compound comprised according to general formula X R^{16a}, R^{16b} and R^{16c} independently of one another represent a linear or branched C₁₋₆-alkyl group, a linear or branched C₁₋₆-alkoxy group, a halogen atom, CH₂F, CHF₂, CF₃, CN, OH, NO₂, - (C=O)-R', SH, SR', SOR', SO₂R', NH₂, NHR', NR'R", -(C=O)-NH₂, -(C=O)-NHR' and -(C=O)-NR'R" whereby R' and R" for each substituent independently represent linear or branched C₁₋₆ alkyl; preferably methyl, ethyl, F, Cl, Br and CF₃, more preferably R^{16a} represents a chlorine atom in the 4-position, whereas R^{16b}.and R^{16c} represent hydrogen.

In a preferred embodiment of the medicament according to the invention for the compound comprised according to general formula X R^{17a}, R^{17b} and R^{17c} independently of one another represent linear or branched C₁₋₆-alkyl group, a linear or branched C₁₋₆-alkoxy group, a halogen atom, CH₂F, CHF₂, CF₃, CN, OH, NO₂, - (C=O)-R', SH, SR', SOR', SO₂R', NH₂, NHR', NR'R", -(C=O)-NH₂, -(C=O)-NHR' and -(C=O)-NR'R", whereby R' and optionally R" for each substituent independently represent linear or branched C₁₋₆ alkyl, preferably R^{17a}, R^{17b} and R^{17c} represent methyl, ethyl, F, Cl, Br and CF3, more preferably R^{17a} and R^{17b} represent two chlorine atoms in 2- and 4-position and R^{17c} represents hydrogen.

In a preferred embodiment of the medicament according to the invention for the compound comprised according to general formula X R¹⁸ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, or an -NR¹⁹R²⁰-moiety, preferably R¹⁸ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or an -NR¹⁹R²⁰-moiety, more preferably R¹⁸ represents a pyrrolidinyl group, a piperidinyl group or a piperazinyl group, whereby each of these groups may be substituted with one or more C₁₋₆-alkyl groups, or an ―NR¹⁸R¹⁹-moiety.

In a preferred embodiment of the medicament according to the invention for the compound comprised according to general formula X R¹⁹ and R²⁰, identical or different, represent a hydrogen atom, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group, an -SO₂-R²¹-moiety, or an -NR²²R²³-moiety, preferably one of these residues R¹⁹ and R²⁰ represents a hydrogen atom and the other one of these residues R¹⁹ and R²⁰ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an - SO₂-R²¹-moiety, or an -NR²²R²³-moiety, or R¹⁹ and R²⁰, identical or different, each represent a C₁₋₆ alkyl group, more preferably one of these residues R¹⁹ and R²⁰ represents a hydrogen atom and the other one of these residues R¹⁹ and R²⁰ represents an optionally at least mono-substituted pyrrolidinyl group, an optionally at least mono-substituted piperidinyl group, an optionally at least mono-substituted piperazinyl group, an optionally at least mono-substituted triazolyl group, an -SO₂-R²¹-moiety, or an -NR²²R²³-moiety, or R¹⁹ and R²⁰, identical or different, represent a methyl group, an ethyl group, an n-propyl group, an isopropyl group, an n-butyl group, a sec-butyl group or a tert.-butyl group.

In a preferred embodiment of the medicament according to the invention for the compound comprised according to general formula X R²¹ represents a linear or branched, saturated or unsaturated, optionally at least mono-substituted C₁₋₆ aliphatic group, a saturated or unsaturated, optionally at least mono-substituted, optionally at least one heteroatom as ring member containing C₃₋₈ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene (-CH₂-) or ethylene (-CH₂-CH₂)-group, preferably R²¹ represents a C₁₋₆-alkyl group, a saturated, optionally at least mono-substituted cycloaliphatic group, which may be condensed with a mono- or polycyclic ring-system, or a phenyl group, which is optionally substituted with one or more C₁₋₆ alkyl groups.

In a preferred embodiment of the medicament according to the invention for the compound comprised according to general formula X R²² and R²³, identical or different, represent a hydrogen atom, an unbranched or branched, saturated or unsaturated, optionally at least mono-substituted C₁₋₆ aliphatic radical, a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably R²² and R²³, identical or different, represent a hydrogen atom or a C₁₋₆ alkyl radical.

In a preferred embodiment of the medicament according to the invention for the compound comprised according to general formula X R⁴², represents a hydrogen atom, an unbranched or branched, saturated or unsaturated, substituted or unsubstituted C₁₋₆ alkyl group; preferably hydrogen, methyl or ethyl.

In a preferred embodiment of the medicament according to the invention for the compound comprised according to general formula X R^{16a}, R^{16b} and R^{16c} independently of each other represent hydrogen, a linear or branched C₁₋₆-alkyl group, a linear or branched C₁₋₆-alkoxy group, a halogen atom, or CF₃; preferably hydrogen, chlorine, bromine, C₁₋₃-alkyl, C₁₋₃-alkoxy, CF₃ or NO₂; most preferably R^{16a}, R^{16b} and R^{16c} independently of each other represent hydrogen, methyl, ethyl, F, Cl, Br and CF₃.

In a preferred embodiment of the medicament according to the invention for the compound comprised according to general formula X R^{17a}, R^{17b} and R^{17c} independently of each other represent hydrogen, a linear or branched C₁₋₆-alkyl group, a linear or branched C₁₋₆-alkoxy group, a halogen atom, or CF₃; preferably hydrogen, chlorine, bromine, C₁₋₃-alkyl, C₁₋₃-alkoxy, CF₃ or NO₂.

In a preferred embodiment of the medicament according to the invention for the compound comprised according to general formula X is represented by a structure wherein,
R^{16a} represents a halogen atom, preferably a chlorine or bromine atom, in its 4-position,
R^{16b} and R^{16c} represent hydrogen,
R^{17a} and R^{17b} represent halogen atoms, preferably chlorine atoms, in its 2- and 4-position,
R^{17c} represent hydrogen,
R¹⁸ represents a pyrrolidinyl group, a piperidinyl group, a piperazinyl group, a homo-piperazinyl group, a morpholinyl group, or an ―NR¹⁹R²⁰-moiety,
R¹⁹ represents a hydrogen atom or a linear or branched C₁₋₆-alkyl group,
R²⁰ represents a linear or branched C₁₋₆ alkyl group, an -SO₂-R²¹-moiety, a pyrrolidinyl group, a piperidinyl group, a piperazinyl group, a homo-piperazinyl group, a morpholinyl group, a triazolyl group, whereby each of the heterocyclic rings may be substituted with one or more, identical or different, C₁₋₆-alkyl groups, and
R²¹ represents a phenyl group, which is optionally substituted with one or more C₁₋₆ alkyl groups, which may be identical or different,
R⁴² in a compound according to general formula X represents a hydrogen atom, an unbranched or branched, saturated or unsaturated, substituted or unsubstituted C₁₋₆ alkyl group, preferably hydrogen, methyl or ethyl,
   optionally in form of a corresponding N-oxide thereof, or a corresponding salt thereof, or a corresponding solvate thereof.

In a preferred embodiment of the medicament according to the invention the compound comprised according to general formula X is selected from:
- 1-(2,4-dichlorophenyl)-5-(4-chlorophenyl)-4-methyl-N-(piperidin-1-yl)-1 H-pyrazole-3-carboxamide;
- 1-(2,4-dichlorophenyl)-5-(4-bromophenyl)-4-methyl-N-(piperidin-1-yl)-1 H-pyrazole-3-carboxamide;
- 1-(2,4-dichlorophenyl)-5-(4-chlorophenyl)-4-ethyl-N-(piperidin-1-yl)-1H-pyrazole-3-carboxamide,
- 1-(2,4-dichlorophenyl)-5-(4-bromophenyl)-4-ethyl-N-(piperidin-1-yl)-1H-pyrazole-3-carboxamide;
- 1-(2,4-dichlorophenyl)-5-(4-chlorophenyl)-N-(piperidin-1-yl)-1H-pyrazole-3-carboxamide or
- 1-(2,4-dichlorophenyl)-5-(4-bromophenyl)-N-(piperidin-1-yl)-1H-pyrazole-3-carboxamide
   optionally in the form of a corresponding N-oxide, a corresponding salt or a corresponding solvate.

In a preferred embodiment of the medicament according to the invention at least one of the compound comprised is selected from:
- 1-(2,4-dichlorophenyl)-5-(4-chlorophenyl)-4-methyl-1H-pyrazole-3-carboxylic acid,
- 1-(2,4-dichlorophenyl)-5-(4-bromophenyl)-4-methyl-1H-pyrazole-3-carboxylic acid,
- 1-(2,4-dichlorophenyl)-5-(4-chlorophenyl)-4-ethyl-1H-pyrazole-3-carboxylic acid,
- 1-(2,4-dichlorophenyl)-5-(4-bromophenyl)-4-ethyl-1H-pyrazole-3-carboxylic acid,
- 1-(2,4-dichlorophenyl)-5-(4-chlorophenyl)-1H-pyrazole-3-carboxylic acid, or
- 1-(2,4-dichlorophenyl)-5-(4-bromophenyl)-1H-pyrazole-3-carboxylic acid
   and
- 1-(2,4-dichlorophenyl)-5-(4-chlorophenyl)-4-methyl-N-(piperidin-1-yl)-1 H-pyrazole-3-carboxamide;
- 1-(2,4-dichlorophenyl)-5-(4-bromophenyl)-4-methyl-N-(piperidin-1-yl)-1 H-pyrazole-3-carboxamide;
- 1-(2,4-dichlorophenyl)-5-(4-chlorophenyl)-4-ethyl-N-(piperidin-1-yl)-1H-pyrazole-3-carboxamide;
- 1-(2,4-dichlorophenyl)-5-(4-bromophenyl)-4-ethyl-N-(piperidin-1-yl)-1H-pyrazole-3-carboxamide;
- 1-(2,4-dichlorophenyl)-5-(4-chlorophenyl)-N-(piperidin-1-yl)-1H-pyrazole-3-carboxamide or
- 1-(2,4-dichlorophenyl)-5-(4-bromophenyl)-N-(piperidin-1-yl)-1H-pyrazole-3-carboxamide
   each optionally in the form of a corresponding N-oxide, a corresponding salt or a corresponding solvate.

Another highly preferred aspect of the invention is the use of a compound of general formula I, la, II, or Ila described above or of a combination of compounds described above of at least one compound according to formulas I, la, II or Ila and at least one compound according to general formula X or Xa for the manufacture of a Medicament for the treatment of the metabolic syndrome.

As defined here treatment does also include prophylaxis.

The Metabolic syndrome is described in detail by Eckel et al., The Lancet, Vol. 365 (2005), 1415-1428, included here by reference. Even though according to Eckel et al. there is no homogemeous definition of definition of the metabolic syndrome it can clearly be derived from the article and the statements of the WHO that metabolic syndrome is to be considered as a disease which can and has to be treated. Especially it involves inter alia (pathophysiological) changes in the blood parameter, especially lipid parameters. Even though obesity plays an important role in the metabolic syndrome some aspects of the syndrome are weight independent, especially some lipid parameters. Especially the positive influence on the weight independent aspects of the metabolic syndrome (see e.g. Pagotto and Pasquali, The Lancet, Vol. 365 (2005), 1363, 1364, included by reference) like some blood parameters, especially lipid parameters, including influence on Triglyceride levels, is one of the major and surprising advantages of the compounds according to general formula I, Ia, II or Ila, and their combination with compounds according to general formula X or Xa. On the other hand given the interrelation between metabolic syndrome and diabetes, especially diabetes type II, glucose intolerance and insulin resistance the compounds do have an influence here. Given the very nature of the metabolic syndrome treatment of it seems also to be beneficial for the treatment of cardiovascular diseases especially cardiovascular risk factors, including prophylaxis. As a last point it thus might also be that the compounds act also in food disorders, including obesity.

Thus in a preferred embodiment of the use according to the invention the metabolic syndrome is weight independent.

Thus in a preferred embodiment of the use according to the invention the medicament is influencing/treating the blood parameters, especially the lipid parameters. It seems that the compounds advantageously lower the proportion of LDL bodies//proteins and raise thye proportion of HDL bodies/proteins.

Thus in a preferred embodiment of the use according to the invention the medicament is for the treatment of diabetes, especially type II, glucose intolerance and insulin resistance.

A preferred aspect of the invention is the use of a compound of general formula I, la, II, or Ila described above or of a combination of compounds described above of at least one compound according to formulas I, la, II or Ila and at least one compound according to general formula X or Xa for the manufacture of a medicament for the treatment of diabetes, especially type II, glucose intolerance and insulin resistance.

Another preferred aspect of the invention is also a method of treatment encompassing abovementioned uses, wherein a compound of general formula I or II, and a combination of these with a compound of general formula X is applied to a person in need thereof, treating metabolic syndrome, especially weight independent, cardiovascular diseases especially fighting cardiovascular risk factors, influencing the blood parameters, especially the lipid parameters, diabetes, especially type II, glucose intolerance and insulin resistance.

A highly preferred embodiment of the invention is drawn to N-Oxides of the compounds according to general formula X as described above, which showed improved attributes when used as pharmaceutically active compounds in the body of a mammal.

Especially preferred are thus the following compounds:
- 1-(2,4-dichlorophenyl)-5-(4-chlorophenyl)-4-methyl-N-(piperidin-1-yl)-1 H-pyrazole-3-carboxamide, N-oxide;
- 1-(2,4-dichlorophenyl)-5-(4-bromophenyl)-4-methy(-N-(piperidin-1-yl)-1 H-pyrazole-3-carboxamide, N-oxide;
- 1-(2,4-dichlorophenyl)-5-(4-chlorophenyl)-4-ethyl-N-(piperidin-1-yl)-1H-pyrazole-3-carboxamide, N-oxide;
- 1-(2,4-dichlorophenyl)-5-(4-bromophenyl)-4-ethyl-N-(piperidin-1-yl)-1H-pyrazole-3-carboxamide, N-oxide;
- 1-(2,4-dichlorophenyl)-5-(4-chlorophenyl)-N-(piperidin-1-yl)-1H-pyrazole-3-carboxamide, N-oxide or
- 1-(2,4-dichlorophenyl)-5-(4-bromophenyl)-N-(piperidin-1-yl)-1H-pyrazole-3-carboxamide, N-oxide.

A thus preferred embodiment encompasses also a medicament comprising at least one of the N-oxide compounds of general formula X or especially at least one of the preferred 6 N-oxides listed above as well as optionally one or more pharmaceutically acceptable excipients.

In a further preferred embodiment at least one of the N-oxide compounds of general formula X or especially at least one of the preferred 6 N-oxides listed above is used for the manufacture of a medicament for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders; for the prophylaxis and/or treatment of food intake disorders, preferably bulimia, anorexia, cachexia, obesity, type II diabetus mellitus (non-insuline dependent diabetes mellitus), more preferably obesity; for the prophylaxis and/or treatment of psychosis; for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction; for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer; metabolic syndrome, especially weight independent, cardiovascular diseases, cardiovascular risk factors, glucose intolerance and insulin resistance; for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Medicaments/drugs, which are frequently the subject of misuse include opioids, barbiturates, cannabis, cocaine, amphetamines, phencyclidine, hallucinogens and benzodiazepines.

Another highly preferred aspect of the invention is the use of the N-Oxides of the compounds according to general formula I or II, the N-oxides X of the compounds according to general formula X as well as the combinations tereof as active principle bound to and/or eluting from a medicinal implant especially a cardiovascular implant, most preferably a stent, especially for the prophylaxis or treatment of restenosis. As - as described above - it seems that the compounds advantageously lower the proportion of LDL bodies//proteins and raise the proportion of HDL bodies/proteins the drug eluting from the stent, bound thereto physically or chemically (covalently or non-covalently), will be positively influencing the effectivity of a cardiovascular stent allowing it a longer time of implantation.

For this embodiment the following compounds - as well as combinations between the respective compounds of dormula I or II and X - are especially preferred:

### (formula I or II:)

• 1-(2,4-dichlorophenyl)-5-(4-chlorophenyl)-4-methyl-1H-pyrazole-3-carboxylic acid,
• 1-(2,4-dichlorophenyl)-5-(4-bromophenyl)-4-methyl-1H-pyrazole-3-carboxylic acid,
• 1-(2,4-dichlorophenyl)-5-(4-chlorophenyl)-4-ethyl-1H-pyrazole-3-carboxylic acid,
• 1-(2,4-dichlorophenyl)-5-(4-bromophenyl)-4-ethyl-1 H-pyrazole-3-carboxylic acid,
• 1-(2,4-dichlorophenyl)-5-(4-chlorophenyl)-1H-pyrazole-3-carboxylic acid, or
• 1-(2,4-dichtorophenyl)-5-(4-bromophenyl)-1H-pyrazole-3-carboxylic acid
   and

### (formula X:)

• 1-(2,4-dichlorophenyl)-5-(4-chlorophenyl)-4-methyl-N-(piperidin-1-yl)-1 H-pyrazole-3-carboxamide;
• 1-(2,4-dichlorophenyl)-5-(4-bromophenyl)-4-methyl-N-(piperidin-1-yl)-1 H-pyrazole-3-carboxamide;
• 1-(2,4-dichlorophenyl)-5-(4-chlorophenyl)-4-ethyl-N-(piperidin-1-yl)-1H-pyrazole-3-carboxamide;
• 1-(2,4-dichlorophenyl)-5-(4-bromophenyl)-4-ethyl-N-(piperidin-1-yl)-1H-pyrazole-3-carboxamide;
• 1-(2,4-dichlorophenyl)-5-(4-chlorophenyl)-N-(piperidin-1-yl)-1H-pyrazole-3-carboxamide or
• 1-(2,4-dichlorophenyl)-5-(4-bromophenyl)-N-(piperidin-1-yl)-1H-pyrazole-3-carboxamide
   each optionally in the form of a corresponding N-oxide, a corresponding salt or a corresponding solvate.

The present invention is illustrated below with the aid of examples. These illustrations are given solely by way of example and are not intended to limit the present invention.

**Examples:**

## Claims

1. Medicament comprising at least one substituted pyrazole compounds of general formula I, 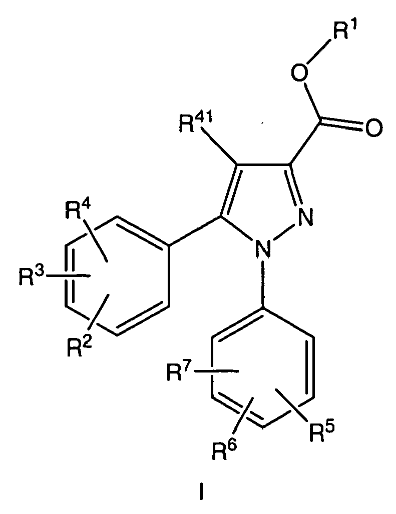 wherein
R¹ represents hydrogen or a linear or branched, substituted or unsubstituted, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵, R⁶ and R⁷independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby
R¹⁰ and optionally R¹¹ for each substituent independently represent linear or branched C₁₋₆ alkyl;
R⁴¹ represents hydrogen, a linear or branched, substituted or unsubstituted, saturated or unsaturated C₁₋₆-alkyl group, a linear or branched, substituted or unsubstituted, saturated or unsaturated C₁₋₆-alkoxy group, a halogen atom, CH₂F, CHF₂, CF₃, CN, OH, NO₂, -(C=O)-R^{10a}, SH, SR^{10a}, SOR^{10a}, NH₂, NHR^{10a}, NR^{10a}R^{11a}, -(C=O)-NH₂, -(C=O)-NHR^{10a} and -(C=O)-NR^{10a}R^{11a}, whereby R^{10a} and optionally R^{11a} for each substituent independently represent linear or branched C₁₋₆ alkyl;
optionally in form of a corresponding N-oxide thereof, or a corresponding salt thereof, or a corresponding solvate thereof,
and optionally one or more pharmaceutically acceptable excipients.

2. Medicament according to claim 1, **characterized in that** in the compound according to general formula I R⁴¹ represents hydrogen or a linear or branched, substituted or unsubstituted, saturated or unsaturated C₁₋₆-alkyl group; preferably hydrogen or C₁₋₃-alkyl; most preferably hydrogen, methyl or ethyl.

3. Medicament according to any of claims 1 or 2, **characterized in that** in the compound according to general formula I at least one of R², R³ or R⁴ represents hydrogen, while at least one of R², R³ or R⁴ is different from hydrogen.

4. Medicament according to any one of claims 1 to 3, **characterized in that** in the compound according to general formula I R², R³ and R⁴ independently of each other represent hydrogen, a linear or branched C₁₋₆-alkyl group, a linear or branched C₁₋₆-alkoxy group, a halogen atom, or CF₃; preferably hydrogen, chlorine, bromine, C₁₋₃-alkyl, C₁₋₃-alkoxy, CF₃ or NO₂; most preferably R², R³ and R⁴ independently of each other represent hydrogen, methyl, ethyl, F, Cl, Br and CF₃.

5. Medicament according to any one of claims 1 to 4, **characterized in that** in the compound according to general formula I R⁵, R⁶ and R⁷ independently of each other represent hydrogen, a linear or branched C₁₋₆-alkyl group, a linear or branched C₁₋₆-alkoxy group, a halogen atom, or CF₃; preferably hydrogen, chlorine, bromine, C₁₋₃-alkyl, C₁₋₃-alkoxy, CF₃ or NO₂.

6. Medicament according to any one of claims 1 to 5, **characterized in that** in the compound according to general formula I R⁷ represents hydrogen.

7. Medicament according to any one of claims 1 to 6, **characterized in that** in the compound according to general formula I R⁵ and R⁶ independently of each other represent a linear or branched C₁₋₆-alkyl group, a halogen atom, or CF₃, preferably R⁵ and R⁶ independently of each other represent methyl, ethyl, F, Cl, Br and CF₃.

8. Medicament according to any one of claims 1 to 7, **characterized in that** in the compound according to general formula I R² represents a chlorine or a bromine atom in the 4-position of the phenyl ring, while R³ and R⁴ represent hydrogen.

9. Medicament according to any one of claims 1 to 8, **characterized in that** in the compound according to general formula I R⁵ and R⁶ each represent a chlorine atoms in the 2- and 4-position of the phenyl ring, while R⁷ represents hydrogen.

10. Medicament according to any one of claims 1 to 9, **characterized in that** in the compound according to general formula I R¹ represents hydrogen, methyl or ethyl, preferably hydrogen.

11. Medicament according to one or more of claims 1 to 10, **characterized in that** the compound according to general formula I is selected from a compound of general formula II 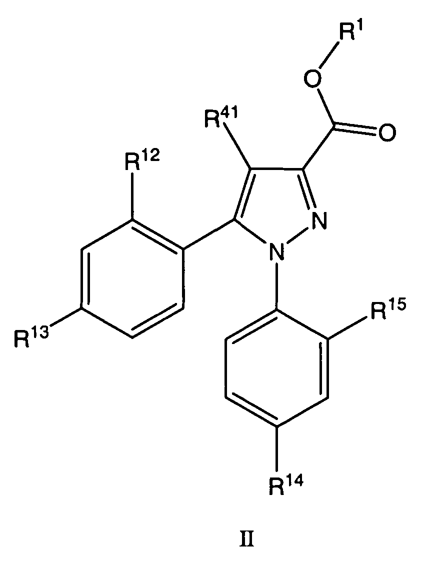 wherein
R¹ represents hydrogen or a linear or branched, substituted or unsubstituted, saturated or unsaturated C₁₋₄-alkyl group,
R¹² in a compound according to general formula II represents hydrogen, a linear or branched, substituted or unsubstituted, saturated or unsaturated C₁₋₆-alkyl group; a linear or branched, substituted or unsubstituted, saturated or unsaturated C₁₋₆-alkoxy group; a halogen atom, CN, OH, NO₂, SH, NH₂,
R¹³ represents a linear or branched, substituted or unsubstituted, saturated or unsaturated C₁₋₆-alkyl group; a linear or branched, substituted or unsubstituted, saturated or unsaturated C₁₋₆-alkoxy group; a halogen atom, CN, OH, NO₂, SH, NH₂,
R¹⁴ or R¹⁵ independently of each other represent a linear or branched, substituted or unsubstituted, saturated or unsaturated C₁₋₆-alkyl group; a linear or branched, substituted or unsubstituted, saturated or unsaturated C₁₋₆-alkoxy group; a halogen atom, CN, OH, NO₂, SH, NH₂,
R⁴¹ represents hydrogen, a linear or branched, substituted or unsubstituted, saturated or unsaturated C₁₋₆-alkyl group; a linear or branched, substituted or unsubstituted, saturated or unsaturated C₁₋₆-alkoxy group; a halogen atom, CN, OH, NO₂, SH, NH₂;
optionally in form of a corresponding N-oxide thereof, or a corresponding salt thereof, or a corresponding solvate thereof.

12. Medicament according to claim 11, **characterized in that** in a compound according to general formula II R⁴¹ represents hydrogen or a linear or branched C₁₋₆-alkyl group, preferably hydrogen, methyl or ethyl.

13. Medicament according to any of claims 11 or 12 **characterized in that** in a compound according to general formula II R¹² represent hydrogen, a linear or branched C₁₋₆-alkyl group, a halogen atom, CH₂F, CHF₂, CF₃, CN, OH, NO₂, SH, or NH₂, preferably R¹² represents hydrogen, methyl, ethyl, F, Cl, Br and CF₃.

14. Medicament according to any of claims 11 to 13 **characterized in that** in the compound according to general formula II R¹³ represents hydrogen, a linear or branched C₁₋₆-alkyl group, a halogen atom, CH₂F, CHF₂, CF₃, CN, OH, NO₂, SH, or NH₂, preferably R¹³ represents hydrogen, methyl, ethyl, F, Cl, Br and CF₃.

15. Medicament according to any one of claims 11 to 14, **characterized in that** in the compound according to general formula II R¹⁴, and R¹⁵ independently of each other represent a linear or branched C₁₋₆-alkyl group, a halogen atom, or CF₃, preferably R¹⁴ and R¹⁵ independently of each other represent methyl, ethyl, F, Cl, Br and CF₃.

16. Medicament according to any one of claims11 to 15, **characterized in that** in a compound according to general formula II R¹² represents hydrogen.

17. Medicament according to any one of claims11 to 16, **characterized in that** in the compound according to general formula II R¹³ represents Cl or Br.

18. Medicament according to any one of claims 11 to 17, **characterized in that** in the compound according to general formula II R¹⁴ and R¹⁵ each represent Cl.

19. Medicament according to any one of claims 11 to 18, **characterized in that** in the compound according to general formula II R¹ represents hydrogen, methyl or ethyl, preferably hydrogen.

20. Medicament according to one or more of claims 1 to 19, **characterized in that** the compound according to general formula I or II is selected from the group consisting of:
• 1-(2,4-dichlorophenyl)-5-(4-chlorophenyl)-4-methyl-1H-pyrazole-3-carboxylic acid,
• 1-(2,4-dichlorophenyl)-5-(4-bromophenyl)-4-methyl-1H-pyrazole-3-carboxylic acid,
• 1-(2,4-dichlorophenyl)-5-(4-chlorophenyl)-4-ethyl-1H-pyrazole-3-carboxylic acid,
• 1-(2,4-dichlorophenyl)-5-(4-bromophenyl)-4-ethyl-1H-pyrazole-3-carboxylic acid
• 1-(2,4-dichlorophenyl)-5-(4-chlorophenyl)-1H-pyrazole-3-carboxylic acid, or
• 1-(2,4-dichlorophenyl)-5-(4-bromophenyl)-1H-pyrazole-3-carboxylic acid,
optionally in the form of a corresponding N-oxide, a corresponding salt or a corresponding solvate.

21. Medicament according to any of claims 1 to 20, comprising at least one compound according to either of general formulas I or II and at least one substituted pyrazole compound according to general formula X, 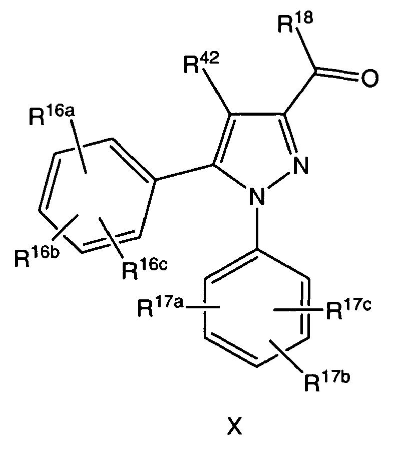 wherein
R^{16a}, R^{16b} and R^{16c} independently of one another represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁶, SH, SR²⁶, SOR²⁶, SO₂R²⁶, NH₂, NHR²⁶, NR²⁶R²⁷, -(C=O)-NH₂, -(C=O)-NHR²⁶ or -(C=O)-NR²⁶R²⁷ whereby R²⁶ and R²⁷ for each substituent independently represent linear or branched C₁₋₆ alkyl,
R^{17a}, R^{17b} and R^{17c} independently of one another represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁶, SH, SR²⁶, SOR²⁶, SO₂R²⁶, NH₂, NHR²⁶, NR²⁶R²⁷, -(C=O)-NH₂, -(C=O)-NHR²⁶ or -(C=O)-NR²⁶R²⁷ whereby R²⁶ and R²⁷ for each substituent independently represent linear or branched C₁₋₆ alkyl,
R¹⁸ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system, or an ― NR¹⁹R²⁰-moiety,
R¹⁹ and R²⁰, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an -SO₂-R²¹-moiety, or an - NR²²R²³-moiety, with the proviso that R¹⁹ and R²⁰ do not identically represent hydrogen,
R²¹ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R²² and R²³, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
R⁴² in the compound according to general formula X represents hydrogen; a linear or branched, saturated or unsaturated, substituted or unsubstituted C₁₋₆-alkyl group; a linear or branched, saturated or unsaturated, substituted or unsubstituted C₁₋₆-alkoxy group, a halogen atom, CH₂F, CHF₂, CF₃, CN, OH, NO₂, -(C=O)-R²⁴, SH, SR²⁴, SOR²⁴, NH₂, NHR²⁴, NR²⁴R²⁵, -(C=O)-NH₂, - (C=O)-NHR²⁴ and -(C=O)-NR²⁴R²⁵, whereby R²⁴ and optionally R²⁵ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

22. Medicament according to claim 21, **characterized in that** in the compound according to general formula X R^{16a}, R^{16b} and R^{16c} independently of one another represent a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and - (C=O)-NR'R" whereby R' and R" for each substituent independently represent linear or branched C₁₋₆ alkyl; preferably methyl, ethyl, F, Cl, Br and CF₃, more preferably R^{16a} represents a chlorine atom in the 4-position, whereas R^{16b}.and R^{16c} represent hydrogen.

23. Medicament according to any of claims 21 or 22, **characterized in that** in the compound according to general formula X R^{17a}, R^{17b} and R^{17c} independently of one another represent linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and - (C=O)-NR'R", whereby R' and optionally R" for each substituent independently represent linear or branched C₁₋₆ alkyl, preferably R^{17a}, R^{17b} and R^{17c} represent methyl, ethyl, F, Cl, Br and CF3, more preferably R^{17a} and R^{17b} represent two chlorine atoms in 2- and 4-position and R^{17c} represents hydrogen.

24. Medicament according to any of claims 21 to 23, **characterized in that** in the compound according to general formula X R¹⁸ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, or an ―NR¹⁹R²⁰-moiety, preferably R¹⁸ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or an ―NR¹⁹R²⁰-moiety, more preferably R¹⁸ represents a pyrrolidinyl group, a piperidinyl group or a piperazinyl group, whereby each of these groups may be substituted with one or more C₁₋₆-alkyl groups, or an ―NR¹⁸R¹⁹-moiety.

25. Medicament according to any of claims 21 to 24, **characterized in that** in the compound according to general formula X R¹⁹ and R²⁰, identical or different, represent a hydrogen atom, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic group, which may be condensed with an optionally at least mono-substituted mono-or polycyclic ring system, or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group, an ―SO₂-R²¹-moiety, or an ―NR²²R²³-moiety, preferably one of these residues R¹⁹ and R²⁰ represents a hydrogen atom and the other one of these residues R¹⁹ and R²⁰ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an ―SO₂-R²¹-moiety, or an ―NR²²R²³-moiety, or R¹⁹ and R²⁰, identical or different, each represent a C₁₋₆ alkyl group, more preferably one of these residues R¹⁹ and R²⁰ represents a hydrogen atom and the other one of these residues R¹⁹ and R²⁰ represents an optionally at least mono-substituted pyrrolidinyl group, an optionally at least mono-substituted piperidinyl group, an optionally at least mono-substituted piperazinyl group, an optionally at least mono-substituted triazolyl group, an ―SO₂-R²¹-moiety, or an ―NR²²R²³-moiety, or R¹⁹ and R²⁰, identical or different, represent a methyl group, an ethyl group, an n-propyl group, an isopropyl group, an n-butyl group, a sec-butyl group or a tert.-butyl group.

26. Medicament according to any of claims 21 to 25, **characterized in that** in the compound according to general formula X R²¹ represents a linear or branched, saturated or unsaturated, optionally at least mono-substituted C₁₋₆ aliphatic group, a saturated or unsaturated, optionally at least mono-substituted, optionally at least one heteroatom as ring member containing C₃₋₈ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene (-CH₂-) or ethylene (-CH₂-CH₂)-group, preferably R²¹ represents a C₁₋₆-alkyl group, a saturated, optionally at least mono-substituted cycloaliphatic group, which may be condensed with a mono-or polycyclic ring-system, or a phenyl group, which is optionally substituted with one or more C₁₋₆ alkyl groups.

27. Medicament according to any of claims 21 to 26, **characterized in that** in the compound according to general formula X R²² and R²³, identical or different, represent a hydrogen atom, an unbranched or branched, saturated or unsaturated, optionally at least mono-substituted C₁₋₆ aliphatic radical, a saturated or unsaturated, optionally at least mono-substituted, optionally at least one heteroatom as ring member containing C₃₋₈ cycloaliphatic group, which may be condensed with an optionally at least mono-substituted mono-or polycyclic ring system, or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably R²² and R²³, identical or different, represent a hydrogen atom or a C₁₋₆ alkyl radical.

28. Medicament according to any of claims 21 to 27, **characterized in that** in the compound according to general formula X R⁴², represents a hydrogen atom, an unbranched or branched, saturated or unsaturated, substituted or unsubstituted C₁₋₆ alkyl group; preferably hydrogen, methyl or ethyl.

29. Medicament according to any one of claims 21 to 28, **characterized in that** in the compound according to general formula X R^{16a}, R^{16b} and R^{16c} independently of each other represent hydrogen, a linear or branched C₁₋₆-alkyl group, a linear or branched C₁₋₆-alkoxy group, a halogen atom, or CF₃; preferably hydrogen, chlorine, bromine, C₁₋₃-alkyl, C₁₋₃-alkoxy, CF₃ or NO₂; most preferably R^{16a}, R^{16b} and R^{16c} independently of each other represent hydrogen, methyl, ethyl, F, Cl, Br and CF₃.

30. Medicament according to any one of claims 21 to 29, **characterized in that** in the compound according to general formula X R^{17a}, R^{17b} and R^{17c} independently of each other represent hydrogen, a linear or branched C₁₋₆-alkyl group, a linear or branched C₁₋₆-alkoxy group, a halogen atom, or CF₃; preferably hydrogen, chlorine, bromine, C₁₋₃-alkyl, C₁₋₃-alkoxy, CF₃ or NO₂.

31. Medicament according to any of claims 21 to 30, **characterized in that** the compound according to general formula X is represented by a structure wherein,
R^{16a} represents a halogen atom, preferably a chlorine or bromine atom, in its 4-position,
R^{16b} and R^{16c} represent hydrogen,
R^{17a} and R^{17b} represent halogen atoms, preferably chlorine atoms, in its 2- and 4-position,
R^{17c} represent hydrogen,
R¹⁸ represents a pyrrolidinyl group, a piperidinyl group, a piperazinyl group, a homo-piperazinyl group, a morpholinyl group, or an -NR¹⁹R²⁰-moiety,
R¹⁹ represents a hydrogen atom or a linear or branched C₁₋₆-alkyl group,
R²⁰ represents a linear or branched C₁₋₆ alkyl group, an -SO₂-R²¹-moiety, a pyrrolidinyl group, a piperidinyl group, a piperazinyl group, a homo-piperazinyl group, a morpholinyl group, a triazolyl group, whereby each of the heterocyclic rings may be substituted with one or more, identical or different, C₁₋₆-alkyl groups, and
R²¹ represents a phenyl group, which is optionally substituted with one or more C₁₋₆ alkyl groups, which may be identical or different,
R⁴² in a compound according to general formula X represents a hydrogen atom, an unbranched or branched, saturated or unsaturated, substituted or unsubstituted C₁₋₆ alkyl group, preferably hydrogen, methyl or ethyl,
optionally in form of a corresponding N-oxide thereof, or a corresponding salt thereof, or a corresponding solvate thereof.

32. Medicament according to any of claims 21 to 31, **characterized in that** the compound according to general formula X is selected from:
• 1-(2,4-dichlorophenyl)-5-(4-chlorophenyl)-4-methyl-N-(piperidin-1-yl)-1 H-pyrazole-3-carboxamide;
• 1-(2,4-dichlorophenyl)-5-(4-bromophenyl)-4-methyl-N-(piperidin-1-yl)-1 H-pyrazole-3-carboxamide;
• 1-(2,4-dichlorophenyl)-5-(4-chlorophenyl)-4-ethyl-N-(piperidin-1-yl)-1H-pyrazole-3-carboxamide,
• 1-(2,4-dichlorophenyl)-5-(4-bromophenyl)-4-ethyl-N-(piperidin-1-yl)-1H-pyrazole-3-carboxamide;
• 1-(2,4-dichlorophenyl)-5-(4-chlorophenyl)-N-(piperidin-1-yl)-1H-pyrazole-3-carboxamide or
• 1-(2,4-dichlorophenyl)-5-(4-bromophenyl)-N-(piperidin-1-yl)-1H-pyrazole-3-carboxamide
optionally in the form of a corresponding N-oxide, a corresponding salt or a corresponding solvate.

33. Medicament according to any of claims 21 to 32, **characterized in that** at least the following compounds are used:
• 1-(2,4-dichlorophenyl)-5-(4-chlorophenyl)-4-methyl-1H-pyrazole-3-carboxylic acid,
• 1-(2,4-dichlorophenyl)-5-(4-bromophenyl)-4-methyl-1H-pyrazole-3-carboxylic acid,
• 1-(2,4-dichlorophenyl)-5-(4-chlorophenyl)-4-ethyl-1H-pyrazole-3-carboxylic acid,
• 1-(2,4-dichlorophenyl)-5-(4-bromophenyl)-4-ethyl-1 H-pyrazole-3-carboxylic acid,
• 1-(2,4-dichlorophenyl)-5-(4-chlorophenyl)-1H-pyrazole-3-carboxylic acid, or
• 1-(2,4-dichlorophenyl)-5-(4-bromophenyl)-1H-pyrazole-3-carboxylic acid
and
• 1-(2,4-dichlorophenyl)-5-(4-chlorophenyl)-4-methyl-N-(piperidin-1-yl)-1 H-pyrazole-3-carboxamide;
• 1-(2,4-dichlorophenyl)-5-(4-bromophenyl)-4-methyl-N-(piperidin-1-yl)-1 H-pyrazole-3-carboxamide;
• 1-(2,4-dichlorophenyl)-5-(4-chlorophenyl)-4-ethyl-N-(piperidin-1-yl)-1H-pyrazole-3-carboxamide;
• 1-(2,4-dichlorophenyl)-5-(4-bromophenyl)-4-ethyl-N-(piperidin-1-yl)-1H-pyrazole-3-carboxamide;
• 1-(2,4-dichlorophenyl)-5-(4-chlorophenyl)-N-(piperidin-1-yl)-1H-pyrazole-3-carboxamide or
• 1-(2,4-dichlorophenyl)-5-(4-bromophenyl)-N-(piperidin-1-yl)-1H-pyrazole-3-carboxamide
each optionally in the form of a corresponding N-oxide, a corresponding salt or a corresponding solvate.

34. Use of a compound of general formula I, or II described in claims 1 to 20 or of a combination of compounds described in claims 21 to 33 of at least one compound according to formulas I or II and at least one compound according to general formula X for the manufacture of a Medicament for the treatment of the metabolic syndrome.

35. Use according to claim 34, **characterized in that** the metabolic syndrome is weight independent.

36. Use according to claim 34 or 35, **characterized in that** the medicament is influencing/treating the blood parameters, especially the lipid parameters.

37. Use according to claim 34, **characterized in that** the medicament is for the treatment of diabetes, especially type II, glucose intolerance and insulin resistance.

38. Use of a compound of general formula I or II described in claims 1 to 20 or of a combination of compounds described in claims 21 to 33 of at least one compound according to formulas I or II and at least one compound according to general formula X for the manufacture of a Medicament for the treatment of diabetes, especially type II, glucose intolerance and insulin resistance.
